# EUROPÄISCHE PATENTANMELDUNG

(11) **EP 0 985 373 A1**
(43) Veröffentlichungstag der Anmeldung: **15.03.2000**
(21) Anmeldenummer: 99117176.0
(22) Anmeldetag: 01.09.1999
(51) Int. Cl.: A61B 5/00, A61B 5/024

(54) **Vorrichtung zur Überwachung und Auswertung menschlicher Körperfunktionen wie Herzfrequenz, Blutdruck etc.**

(30) Priorität: 11.09.1998 DE 29816366 U
(71) Anmelder: Cvetkovic, Stevo, 46286 Dorsten (DE)
(72) Erfinder: Cvetkovic, Stevo, 46286 Dorsten (DE)
(74) Vertreter: Nunnenkamp, Jörg, Dr.

(57) **Zusammenfassung**

Gegenstand der Erfindung ist eine Vorrichtung zur Überwachung eines menschlichen Probanden durch Auswertung seiner ruhe- und/oder bewegungsabhängigen Körperfunktionen wie Herzfrequenz, Blutdruck, Atemluftmenge, Hautwiderstand etc., insbesondere Handgelenk-Blutdruckcomputer, mit einer Meßeinrichtung (1) zur Erfassung von Meßwerten der Körperfunktion(en), und mit einer Auswerteeinheit (2) zur Speicherung und Bewertung der Meßwerte. Um auf eine entsprechende Bedienperson oder einen Probanden bei Erreichen des Ruhr-/Schlafzustandes Einfluß nehmen zu können ist weiter vorgesehen, daß die Auswerteeinheit (2) Signale an eine Meldeeinrichtung (4) abgibt, wobei die Meßwerte mit Blick auf mögliche Ruhe- und/oder Schlafzustände des Probanden ausgewertet werden, und wobei in Abhängigkeit von der Dauer eines solchen Ruhe- und/oder Schlafzustandes die Meldeeinrichtung (4) entsprechend aktiviert wird.

## Beschreibung

Die Erfindung betrifft eine Vorrichtung zur Überwachung eines menschlichen Probanden durch Auswertung seiner ruhe- und/oder bewegungsabhängigen Körperfunktionen wie Herzfrequenz, Blutdruck, Atemluftmenge, Hautwiderstand etc, insbesondere Handgelenk-Blutdruckcomputer, mit einer Meßeinrichtung zur Erfassung von Meßwerten der Körperfunktion(en), und mit einer Auswerteeinheit zur Speicherung und Bewertung der Meßwerte.

Derartige Vorrichtungen zur Messung des Blutdruckes und/oder der Herzfrequenz sind aus der Praxis bekannt. In diesem Zusammenhang werden in der Literatur sogenannte Handgelenk-Blutdruckcomputer beschrieben. Diese erinnern an eine große Armbanduhr und weisen üblicherweise eine armrundvorgeformte Manschette mit Klettverschluß auf. Nach dem Drücken einer Starttaste werden Blutdruck und Herzfrequenz automatisch gemessen, wobei entsprechende Meßwerte über ein Display mitgeteilt werden. Außerdem ist eine Speicherung der Meßwerte (systolischer und diastolischer Blutdruck) unter Mittelwertbildung möglich.

Dabei wird regelmäßig nach der sogenannten oszillometrischen Methode gearbeitet. In diesem Zusammenhang werden von Pulswellen verursachte Schwingungen der Blutdruckgefäßwände als Druckschwankungen in der um den Arm oder das Handgelenk geschlungenen aufblasbaren Manschette elektronisch registriert. Im einzelnen arbeitet der Blutdruckcomputer im allgemeinen so, daß nach dem Einschalten die Manschette selbsttätig aufgepumpt wird und die Luft später wieder abläßt. - Jedenfalls können auf einfache und bewährte Weise ruhe- und/oder bewegungsabhängige Körperfunktionen wie Herzfrequenz und Blutdruck gemessen werden.

Die vorgenannten Körperfunktionen ändern sich regelmäßig bei Erreichen eines Ruhe- und/oder Schlafzustandes, und zwar in charakteristischer Art und Weise. Beispielsweise verringert sich beim Schlafen die Herzfrequenz. Gleichzeitig sinkt der Blutdruck und wegen der verringerten Stoffwechselvorgänge reduziert sich zugleich auch die Atemluftmenge. Im Vergleich zu einem Menschen im aktiven Zustand steigt darüber hinaus der Hautwiderstand, weil weniger Streßhormone produziert werden, somit die Transpirationsneigung abnimmt.

Bei Fahrzeugführern, Zugführern, Flugzeugführern usw. besteht ein ständiges Problem darin, daß zum Beispiel durch Übermüdung Fehlverhalten zu beobachten ist. So kennt man insbesondere bei LKW-Fahrern den berüchtigten "Sekunden-Schlaf", welcher bereits Ursache zahlreicher Unfälle war. - Hier setzt die Erfindung ein.

Der Erfindung liegt das technische Problem zugrunde, eine derartige Vorrichtung so weiterzubilden, daß auf eine entsprechende Bedienperson oder einen Probanden bei Erreichen des Ruhe-/Schlafzustandes Einfluß genommen werden kann.

Zur Lösung dieser Aufgabe ist eine gattungsgemäße Vorrichtung zur Überwachung eines menschlichen Probanden durch Auswertung seiner ruhe- und/oder bewegungsabhängigen Körperfunktionen dadurch gekennzeichnet, daß die Auswerteeinheit Signale an eine Meldeeinrichtung abgibt, wobei die Meßwerte (in der Auswerteeinheit und/oder in der Meldeeinrichtung) mit Blick auf mögliche Ruhe- und/oder Schlafzustände des Probanden ausgewertet werden, und wobei in Abhängigkeit von der Dauer eines solchen Ruhe- und/oder Schlafzustandes die Meldeeinrichtung entsprechend aktiviert wird. Dabei kann die Meldeeinrichtung in die Vorrichtung integriert und/oder als hiervon unabhängiges Bauteil ausgeführt sein. Weiter sieht die Erfindung vor, daß die aktivierte Meldeeinrichtung bevorzugt ein optisches und/oder akustisches Warnsignal abgibt und/oder mittels eines (geringfügigen) elektrischen Schlages eine Warnfunktion für den Probanden ausübt. Mit anderen Worten kann es sich bei der Meldeeinrichtung um eine in die regelmäßig am Handgelenk angebrachte Vorrichtung integrierte Lampe oder einen Lautsprecher (gegebenenfalls mit zusätzlicher Elektronik) handeln. Gleichzeitig oder alternativ ist es möglich, über innenseitig am Armband vorgesehene Elektroden einen (leichten) elektrischen Schlag auf die Bedienperson auszuüben. Mit Hilfe dieser Elektrode besteht im Rahmen der Erfindung auch die Möglichkeit, den Hautwiderstand (zusätzlich oder alternativ zu den weiteren Körperfunktionen) auszuwerten.

Derartiges wird im allgemeinen so durchgeführt, daß die Auswerteeinheit den zeitlichen Verlauf der Meßwerte der Körperfunktionen ermittelt und (nach der Zeit) differenziert, wobei ab Erreichen vorgegebener Zeit-Gradienten innerhalb wählbarer Zeitspannen die Meldeeinrichtung aktiviert wird. Das heißt, sobald beispielsweise die Herzfrequenz innerhalb einer vorgegebenen Zeitspanne einen bestimmen Abfall aufweist, wird die Meldeeinrichtung aktiviert. Ähnliches geschieht, wenn der Blutdruck in der vorgegebenen Zeitspanne mehr oder minder stark sinkt. In diesem Zusammenhang lassen sich charakteristische Werte für die jeweiligen Zeit-Gradienten ermitteln, die von Bedienperson zu Bedienperson variieren können. So ist es denkbar, daß der Übergang vom Wach- zum Schlafzustand bei dem zu überwachenden Probanden zunächst über mehrere Zyklen beobachtet und abgespeichert wird, so daß charakteristische und individuelle Zeitverläufe der zugehörigen Meßwerte ermittelbar sind. Derartiges ist im Rahmen einer sogenannten Einführungs- oder Testphase denkbar, wenn der Proband die erfindungsgemäße Vorrichtung Tag und Nacht trägt. - Jedenfalls lassen sich charakteristische Zeitverläufe der Meßwerte abspeichern und ermitteln, insbesondere für den kritischen Bereich beim Übergang vom Wach- zum Schlafzustand. Aus diesen Meßwertverläufen können Grenzwerte für die Zeit-Gradienten abgeleitet werden. Sobald diese Grenzwerte innerhalb der gegebenen Zeitspannen überschritten werden, wird die Meldeeinrichtung entsprechend aktiviert.

Weiter sieht die Erfindung in diesem Zusammenhang vor, daß die Meldeeinrichtung in ein Triebwerk integriert ist und/oder auf ein solches einwirkt und zusätzlich zur Darstellung des Warnsignals und/oder der Warnfunktion bei Vorliegen eines Ruhe- oder Schlafzustandes des Probanden in das Triebwerk im Sinne einer Leistungsreduzierung eingreift. Unter dem Strich soll hier also so verfahren werden, daß bei Erreichen der zuvor beschriebenen Grenzwerte der Zeit-Gradienten die Meldeeinrichtung das Triebwerk entsprechend beeinflußt. Denkbar ist es hier beispielsweise, daß in einem Kraftfahrzeug das (heutzutage zumeist realisierte elektronische) Gaspedal zurückgenommen wird, so daß sich automatisch die Geschwindigkeit verlangsamt. Entsprechende Eingriffe sind bei einem Zugtriebwerk oder auch Flugzeugtriebwerk denkbar. Dabei kann die Meldeeinrichtung mehrfach, zum Beispiel in vorgegebenen Zeitabständen, ein Warnsignal abgeben und/oder die Warnfunktion ausüben, damit auf jeden Fall die entsprechende Wirkung (beim Bediener) erreicht wird.

Beispielsweise zur Speicherung der Meßwerte sowie zur Ermittlung der Zeit-Gradienten und zur Ansteuerung der Meldeeinrichtung ist üblicherweise ein Rechner, insbesondere Mikrocomputer, in der Auswerteeinheit vorgesehen. Dementsprechend ist mit geringen Baukosten zu rechnen. Außerdem läßt sich die Meldeeinrichtung vorzugsweise drahtlos von der Auswerteeinheit ansteuern, und zwar zum Beispiel für den Fall, daß die Meldeeinrichtung als von der Vorrichtung unabhängiges Bauteil ausgeführt ist. Hier ist es denkbar, die Meldeeinrichtung im Kraftfahrzeug an beliebiger Position anzubringen, wobei eine entsprechende Auslösung von der Auswerteeinheit drahtlos, über Funk oder IR-Wellen, erfolgt. Eine Nachrüstung ist dementsprechend problemlos möglich. Eine besonders kompakte und preisgünstige Ausführung ist dadurch gekennzeichnet, daß eine integrierte Stromversorgung mit Akkumulator, gegebenenfalls Solarzellen, Aufziehwerk, Bewegungsenergiespeicher etc. vorgesehen ist. Das Aufziehwerk kann im einfachsten Fall wie eine aufziehbare Uhr arbeiten. Denkbar ist es natürlich auch, auf Bewegungsenergiespeicher dergestalt zurückzugreifen, welche natürliche Bewegungen am Handgelenk in gespeicherte Energie umsetzen, wie dies u. a. bei automatischen (mechanischen) Armbanduhren geschieht. Jedenfalls kann auf diese Weise die Energieversorgung für die beschriebene Vorrichtung gleichsam autark gestaltet werden, so daß Fehlfunktionen und/oder ein Ausfall nicht zu befürchten sind.

Im Ergebnis ist im Rahmen der Erfindung ein deutlicher Sicherheitsgewinn zu verzeichnen. Denn die beschriebene Vorrichtung läßt sich problemlos am Handgelenk positionieren und stört während normaler (Fahr-) Tätigkeiten nicht. Die entsprechenden Daten zu den Körperfunktionen werden laufend ausgewertet, so daß zuverlässig Übermüdungserscheinungen registriert werden. Gleichzeitig lassen sich in Abhängigkeit von solchen Meßergebnissen Gegenmaßnahmen einleiten, die nicht nur darin bestehen, die Bedienperson bzw. den Probanden zu warnen, sondern gleichzeitig in das Fahrzeug im Sinne einer Geschwindigkeitsverringerung eingreifen können. Selbstverständlich ist es auch möglich, die Meldeeinrichtung als Überwachungseinrichtung dergestalt auszuführen, daß beispielsweise von einer zentralen Leitstelle aus die einzelnen Fahrer überwacht und gegebenenfalls Gegenmaßnahmen ergriffen werden. Dabei kann auch so vorgegangen werden, daß die Meldeeinrichtung entsprechende Daten an die hiervon unabhängige Überwachungseinrichtung weitergibt. Jedenfalls erlaubt die Erfindung eine zuverlässige Überwachung und Kontrolle von insbesondere Fahrzeugführern, die darüber hinaus auf drohende Schlafzustände aufmerksam gemacht werden. Dies alles führt zu einer erheblichen Verbesserung der Sicherheit, welche bisher nicht erreicht werden konnte. Hierin sind die wesentlichen Vorteile der Erfindung zu sehen.

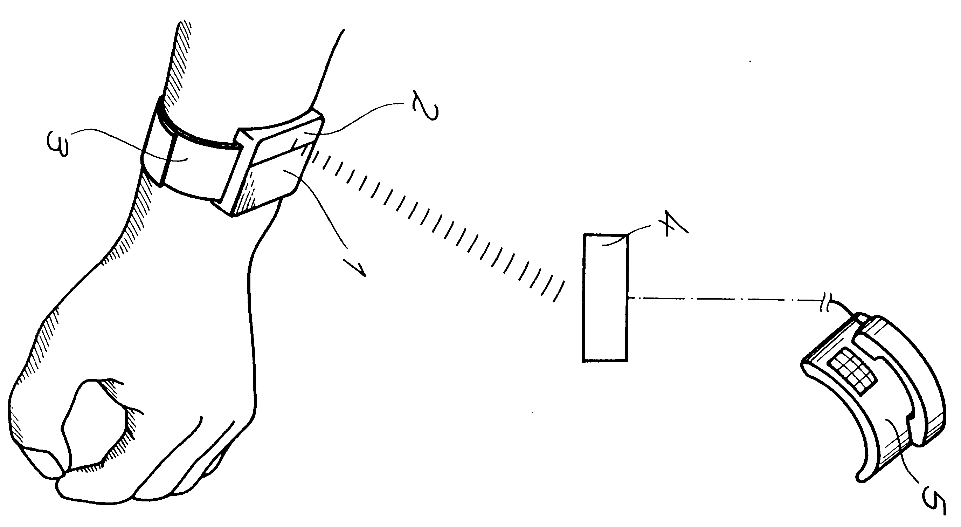

Im folgenden wird die Erfindung anhand einer lediglich ein Ausführungsbeispiel darstellenden Zeichnung näher erläutert. Die einzige Figur zeigt eine Vorrichtung zur Überwachung eines menschlichen Probanden durch Auswertung seiner ruhe- und/oder bewegungsabhängigen Körperfunktionen in schematischer Darstellung.

Im einzelnen ist eine Vorrichtung zur Überwachung eines menschlichen Probanden dargestellt, von welchem lediglich das Handgelenk gezeigt ist. Die vorgenannte Vorrichtung wertet die ruhe- und/oder bewegungsabhängigen Körperfunktionen des Probanden aus. Bei diesen Körperfunktionen kann es sich um die Herzfrequenz, den Blutdruck, die Atemluftmenge, den Hautwiderstand etc. handeln. Vorliegend wird ein sogenannter Handgelenk-Blutdruckcomputer mit Meldefunktion verfolgt. Dieser weist in seinem grundsätzlichen Aufbau eine Meßeinrichtung 1 sowie eine Auswerteeinheit 2 zur Speicherung und Bewertung der Meßwerte auf. Meßeinrichtung 1 und Auswerteeinheit 2 sind an ein Armband 3 mit Klettverschluß angeschlossen. Dieses Armband 3 bildet eine armrundvorgeformte Manschette, welche sich zur Blutdruckmessung selbsttätig aufpumpt und die Luft später wieder abläßt. Hierdurch läßt sich der Blutdruck nach der in der Einleitung beschriebenen oszillometrischen Methode ermitteln. Gleichzeitig ist eine Überwachung der Herzfrequenz möglich. Beide Körperfunktionen werden entweder durchgängig oder periodisch überprüft.

Die Auswerteeinheit 2 gibt Signale an eine Meldeeinrichtung 4 ab. In ihr (das heißt in der Auswerteeinheit 2) werden die ermittelten Meßwerte mit Blick auf mögliche Ruhe- und/oder Schlafzustände des Probanden ausgewertet. In Abhängigkeit von der Dauer eines solchen Ruhe- und/oder Schlafzustandes wird die Meldeeinrichtung 4 entsprechend aktiviert. Sie, das heißt die Meldeeinrichtung 4, läßt sich in die gezeigte Vorrichtung integrieren oder ist - wie dargestellt - als hiervon unabhängiges Bauteil ausgeführt. Darüber hinaus kann sie ein optisches und/oder akustisches Warnsignal abgeben. Zu diesem Zweck läßt sich in die Meldeeinrichtung 4 eine Lampe und/oder ein Lautsprecher integrieren. Gleichzeitig besteht die Möglichkeit, mittels eines elektrischen Schlages eine Warnfunktion für den Probanden auszuüben. Dies erfolgt über eine nicht dargestellte Elektrode, die am Handgelenk angebracht ist und mit einer Spannung im Bereich von ca. 30 bis 40 Volt beaufschlagt wird. Das heißt, in diesem Fall ist die Meldeeinrichtung 4 gleichsam zweigeteilt und besteht aus der am Handgelenk vorgesehenen Elektrode mit zugehöriger Spannungseinrichtung sowie dem externen Bauteil.

Generell ermittelt die Auswerteeinheit 2 den zeitlichen Verlauf der vorgenannten Meßwerte, das heißt den Verlauf der Herzfrequenz und/oder des Blutdruckes über die Zeit. Entsprechende Bewertungen können natürlich prinzipiell (alternativ oder zusätzlich) auch in der Meldeeinrichtung 4 vorgenommen werden. Diese Zeitverläufe werden (nach der Zeit) differenziert, wobei ab Erreichen vorgegebener Zeit-Gradienten innerhalb wählbarer Zeitspannen die Meldeeinrichtung 4 aktiviert wird. Grenzwerte für diese Zeit-Gradienten können individuell festgelegt werden, wie dies bereits beschrieben wurde. Darüber hinaus kann die gezeigte Meldeeinrichtung 4 in ein Triebwerk integriert sein, was jedoch nicht dargestellt ist. Auf diese Weise besteht die Möglichkeit, zusätzlich zur Darstellung des Warnsignals und/oder der Warnfunktion bei Vorliegen eines Ruhe- oder Schlafzustandes in das entsprechende Triebwerk im Sinne einer Leistungsreduzierung einzugreifen. Im einfachsten Fall geschieht dies dergestalt, daß bei einem Verbrennungsmotor einfach das zugehörige, zumeist vorgesehene elektronische, Gaspedal in Richtung auf den Leerlauf verstellt wird.

Die Meldeeinrichtung 4 kann das vorgenannte Warnsignal mehrfach, zum Beispiel in vorgegebenen Zeitabständen, aussenden. Gleiches gilt für die Warnfunktion. Die Zeitabstände lassen sich hierbei periodisch oder auch zufällig bzw. mit sinkendem oder steigendem Zeitabstand einstellen. Schließlich ist in der Auswerteeinheit 2 ein ebenfalls nicht explizit dargestellter Rechner, insbesondere Mikrocomputer, vorgesehen. Dieser dient beispielsweise zur Speicherung der Meßwerte, zur Ermittlung der Zeit-Gradienten und Ansteuerung der Meldeeinrichtung 4. Nach dem Ausführungsbeispiel ist die Meldeeinrichtung 4 drahtlos von der Auswerteeinheit 2 ansteuerbar, wobei übliche Übermittlungstechniken zum Einsatz kommen. Beispielsweise ist eine Übertragung per Infrarotlicht denkbar. Sicherer ist es jedoch, mit Funkwellen im Megaherzbereich zu arbeiten. An die Meldeeinrichtung 4 kann eine Überwachungseinrichtung 5, im Ausführungsbeispiel ein Telefonapparat 5, angeschlossen sein.

Schließlich kann eine integrierte Stromversorgung mit Akku, gegebenenfalls Solarzellen und Aufziehwerk als Energiespeicher vorgesehen sein. Denkbar ist auch die Verwirklichung eines Bewegungsenergiespeichers, wie er bei automatischen mechanischen Uhren bekannt ist. Jedenfalls läßt sich hierdurch eine autarke Energieversorgung der Auswerteeinheit 2 und der Meßeinrichtung 1 erreichen. Dies ist im einzelnen nicht dargestellt.

## Patentansprüche

1. Vorrichtung zur Überwachung eines menschlichen Probanden durch Auswertung seiner ruhe- und/oder bewegungsabhängigen Körperfunktionen wie Herzfrequenz, Blutdruck, Atemluftmenge, Hautwiderstand etc., insbesondere Handgelenk-Blutdruckcomputer, mit einer Meßeinrichtung (1) zur Erfassung von Meßwerten der Körperfunktion(en), und mit einer Auswerteeinheit (2) zur Speicherung und Bewertung der Meßwerte, **dadurch gekennzeichnet,** daß die Auswerteeinheit (2) Signale an eine Meldeeinrichtung (4) abgibt, wobei die Meßwerte mit Blick auf mögliche Ruhe- und/oder Schlafzustände des Probanden ausgewertet werden, und wobei in Abhängigkeit von der Dauer eines solchen Ruhe- und/oder Schlafzustandes die Meldeeinrichtung (4) entsprechend aktiviert wird.

2. Vorrichtung nach Anspruch 1, dadurch gekennzeichnet, daß die Meldeeinrichtung (4) in die Vorrichtung integriert ist und/oder als hiervon unabhängiges Bauteil ausgefuhrt ist.

3. Vorrichtung nach Anspruch 1 oder 2, dadurch gekennzeichnet, daß die aktivierte Meldeeinrichtung (4) ein optisches und/oder akustisches Warnsignal abgibt und/oder mittels eines elektrischen Schlages eine Warnfunktion für den Probanden ausübt.

4. Vorrichtung nach einem der Ansprüche 1 bis 3, dadurch gekennzeichnet, daß die Auswerteeinheit (2) den zeitlichen Verlauf der Meßwerte ermittelt und differenziert, wobei ab Erreichen vorgegebener Zeit-Gradienten innerhalb wählbarer Zeitspannen die Meldeeinrichtung (4) aktiviert wird.

5. Vorrichtung nach einem der Ansprüche 1 bis 4, dadurch gekennzeichnet, daß die Meldeeinrichtung (4) in ein Triebwerk integriert ist und/oder auf ein solches einwirkt und zusätzlich zur Darstellung des Warnsignals und/oder der Warntunktion bei Vorliegen eines Ruhe- und/oder Schlafzustandes in das Triebwerk im Sinne einer Leistungsreduzierung eingreift.

6. Vorrichtung nach einem der Ansprüche 1 bis 5, dadurch gekennzeichnet, daß die Meldeeinrichtung (4) mehrfach, zum Beispiel in vorgegebenen Zeitabständen, ein Warnsignal abgibt und/oder die Warnfunktion ausübt.

7. Vorrichtung nach einem der Ansprüche 1 bis 6, dadurch gekennzeichnet, daß ein Rechner, insbesondere Mikrocomputer, in der Auswerteeinheit (2) beispielsweise zur Speicherung der Meßwerte, zur Ermittlung der Zeit-Gradienten und zur Ansteuerung der Meldeeinrichtung (4) vorgesehen ist.

8. Vorrichtung nach einem der Ansprüche 1 bis 7, dadurch gekennzeichnet, daß die Meldeeinrichtung (4) drahtlos von der Auswerteeinheit (2) ansteuerbar ist.

9. Vorrichtung nach einem der Ansprüche 1 bis 8, dadurch gekennzeichnet, daß eine integrierte Stromversorgung mit Akkumulator, gegebenenfalls Solarzellen, Aufziehwerk, Bewegungsenergiespeicher etc. vorgesehen ist.
